# EUROPEAN PATENT APPLICATION

(11) **EP 2 305 634 A1**
(43) Date of publication of application: **06.04.2011**
(21) Application number: 09839039.6
(22) Date of filing: 16.11.2009
(51) Int. Cl.: C07C 209/14, C07C 209/16, C07C 209/18, C07C 317/06

(54) **THE NEW PREPARATION OF ALIPHATIC AMINES WITH SULPHONYL GROUP AND THEIR SALTS**

(30) Priority: 12.05.2009 CN 200910057216; 03.08.2009 CN 200910057694; 03.08.2009 CN 200910057693
(71) Applicant: Knowa Pharmaceutical (Shanghai) Co. Ltd., Shanghai 201203 (CN)
(72) Inventor: HE, Yong, Shanghai 201203 (CN)
(74) Representative: Schaad, Balass, Menzl & Partner AG
(86) International application number: PCT/CN2009/074958
(87) International publication number: WO 2010/130124

(57) **Abstract**

The present invention relates to a novel preparation method of sulfonyl alkylamine and salts thereof. The method of present invention comprises steps of: adopting sulfonates containing a sulfone moiety as starring material and ammonia oz amine as substituting agent which reacts with the starting material to prepare for the products. Furthermore, the present invention provides a new intermediate 2-(N,N- dibenzylamino) ethyl methyl sulfone and salts thereof obtained from the above novel preparation method and their use. In addition, the present invention also provides a novel salt of 2-(anuno) ethyl methyl sulfone prepared using 2-(amine) ethyl methyl sulfone hydrochloride and the use of novel salt. The present invention is not only advantageous for reducing environmental pollution, but also could be used for mass fabrication of 2-(amino) ethyl methyl sulfone and the salts thereof, in addition, the present invention is advantageous for preparing for the highly purified 2-(amine) ethyl methyl sulfone hydrochloride.

## Description

### BACKGROUND OF THE INVENTION

### 1. Technical Field:

The present invention relates to a novel preparation method of sulfonyl alkylamine which is used as a pharmaceutical intermediate and the salts thereof, N,N-bis(substituted benzyl)-2₋methylsulfonyl ethanamine (that is 2-(N,N- dibenzylamino) ethyl methyl sulfone) which is obtained using this preparation method, its salts and their use. In addition, the present invention also relates to the novel salts of 2-(amino) ethyl methyl sulfone prepared using sulfonyl alkylamine or its hydrochloride salt.

### 2. Description of Related Art:

The sulfonyl alkylamine and its salts are used as important pharmaceutical intermediates for producing some compositions having pharmaceutical activity. 2- methylsulfonyl ethanamine (that is 2-(amino) ethyl methyl sulfone) is one of the essential pharmaceutical intermediates of an anti-tumor component. The reported synthetic route of 2-methylsulfonyl ethanamine is as following: 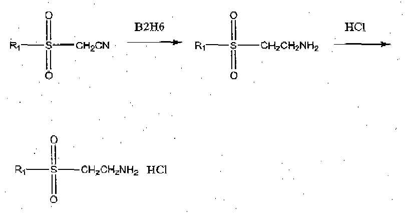 2-methylsulfonyl ethanamine hydrochloride will be obtained via the above synthetic route. However, there are many disadvantages in this route. For example, the diborane used in this route as chemoselective reduction reagent is costly, the hydrochloride salt is hard to be purified and the preparation process is difficult to be applied to the large volume industrial production.

### SUMMARY OF THE INVENTION

The first object of the present invention is to provide a novel preparation method of sulfonyl alkylamine and salts thereof.

The second object of the present invention is to provide a new intermediate 2-(N,N-dibenzylamino) ethyl methyl sulfone (that is N,N-bis(substituted benzyl)-2-methylsulfonyl ethanamine) which is obtained from the said preparation method and its salts.

The third object of the present invention is to provide a novel salt of 2-(aznino) ethyl methyl sulfone (that is the novel salt of 2-methylsulfnnyl ethanamine) prepared by using 2-(amino) ethyl methyl sulfone or its hydrochloride salt (that is 2-(methylsulfonyl)ethanamine hydrochloride.

The present invention provides a novel preparation method of sulfonyl alkylamine and the salts thereof including the steps as following:
A. adopting sulfonates containing a sulfone moiety having following general formula as starting material: 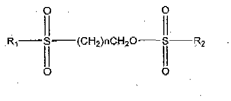 R₁=alkyl
   R₂=Alkyl, Aryl
   n=1∼18
B: adopting ammonia or amine having following general formula as substituting agent to react with the starting material, 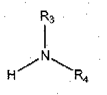 R₃=H, Methyl, Ethyl, n-Propyl
   R₄=H,(CH₂)nR₅ R₅=Alkyl, Aryl, etc n=O or I

The ammonia or amine is used as substituting agent in step B to prepare for the amines and the process is shown as following:

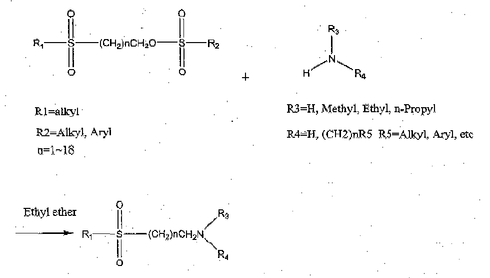

The amine obtained from the substitution reaction above could be further adopted to react with acids to farm salts.

The substitution reaction could be accomplished at room temperature and atmospheric pressure.

In the substitution reaction, the solvent could be ammonia or amine themselves, or the organic solution obtained by dissolving ammonia or amine in alcohol, chloroform, dichloromethane or other organic solvents. The ratio of sulfonates containing a sulfone moiety and the substituting agent selcted from ammonia or amine is as following: the amount of the ammonia or amine is more than twice of the theory amount.

The solvent adopted in the salt-forming reaction is selected from a group consisting of linear or cyclic ether, alcohol, etc.

The salt-foraimg reaction is carried out at atmospheric pressure and room temperature.

The said acid is selected from a group consisting of hydrochloric acid, sulfuric acid, phosphoric acid, boric acid, sulfonic acid or substitute sulfonic acid such as para-toluenestilfonic acid, etc.

As recited in the embodiment of the present invention, the material in step A above is 2'-(methyl sulfone) ethyl methylbenzenesulfonate, the substituting agent is ammonia, the solvent adopted in the reaction is diethyl ether. The solvent adopted in the salt-fomiittg reaction is ethanol.

The corresponding sulfonyl alkylamine and novel salts thereof will be obtained via the preparation method above. The cost of the synthesis route of 2'-(methyl sulfone) ethanamine hydrochloride as above is lower than the cost of the synthesis route reported from the reduction of 2'-(methyl sulfone) acetonitrile.

Furthermore, above-mentioned process can be used to prepare for 2-(N,N- dibenzylamino) ethyl methyl sulfone and the salts thereof.

The chemical structures of the 2-(N,N- dibenzylamino) ethyl methyl sulfone and the salts thereof arc as following: 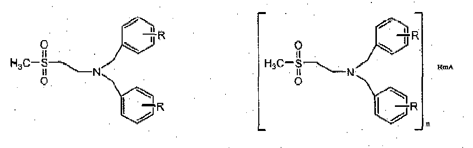 R=H, halogen, alkoxy, etc.
m=1-3, n=3-1
HmA= hydrochloric acid, sulfuric acid, phosphoric acid, etc.

The preparation process of 2-(N,N- dibenzylamino) ethyl methyl sulfone includes the following steps:
A. adopting 2'-(methyl sulfone) ethyl methylbenzenesulfonate having the following chemical structure as starting material: 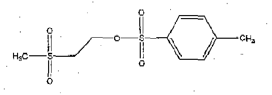
B. adopting N,N-dibenzylamine having the following chemical structure as substituting agent to react with the starting material:

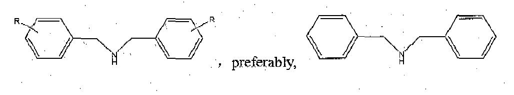

In step B, N,N-dibenzylamine is adopted as substituting agent for preparation of amine which will react with acid (HmA) to form salts in ethanol afterwards and the process is as following: 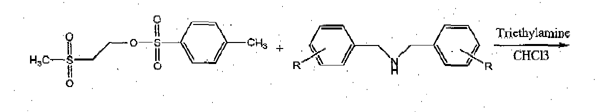 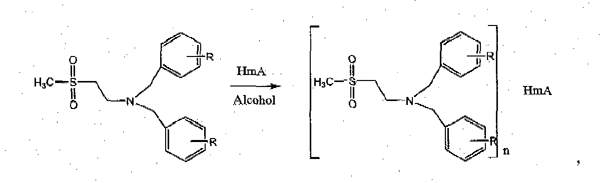 preferably,

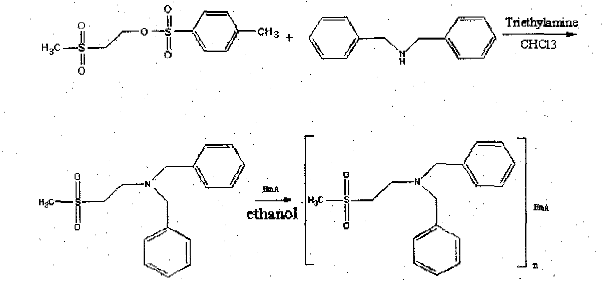

The said substituting reaction could be accomplished at the room temperature and atmospheric pressure.

The said 2-(N,N-dibenzylamino) ethyl methyl sulfone and the salts thereof could be used for the preparation of 2-(amino) ethyl methyl sulfone and its salts via catalytic hydrogenation conveniently.

The preparation process of 2-(amlno) ethyl methyl sulfone and the salts thereof using 2-(N,N-dibeztzylamino) ethyl methyl sulfone and the salts thereof comprises steps as following:
adding 2-(N,N-dibcnzylamino) ethyl methyl sulfone or its salts into the organic solvent selected from a group consisting of alcohols and ethers or the alkaline solutions thereof, then adding Raney Ni, Pd/C or platinum dioxide into the said organic solvent to obtain 2-(amino) ethyl methyl sulfone and the salts thereof after hydrogenated reduction under certain pressure. The process of the reaction is as following: 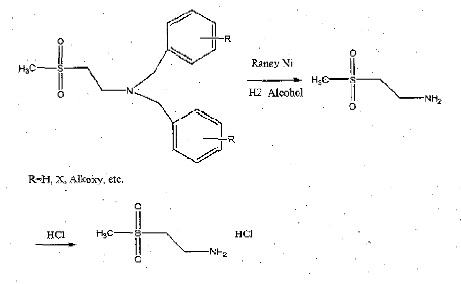 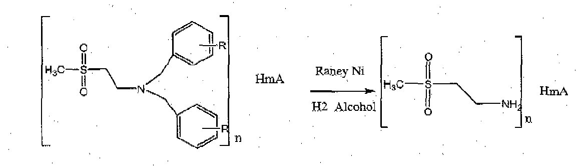
R=H, X, Alkoxy, etc.
m=1-3, n=3-1
HmA= hydrochloric acid, sulfuric acid, phosphoric acid, etc.

Therefore, different salts of 2-(amino) ethyl methyl sulfone will be obtained.

The alkaline solutions of the said organic solvent such as alcohols or ethers are prepared by dissolving sodium hydroxide in the ethanol or dissolving organic base such as triethylamine in the ethanol or diethyl ether.

The said Raney Ni, Pd/C or platinum dioxide is used as the catalytic agent in the catalytic hydrogenation and the addition amount thereof is ranging from 1% to 20% of the reactant amount.

In general, the reaction conditions of the hydrogenated reduction are as follows the temperature is ranging from 10°C to 90°C, the pressure is ranging from 5 to 40 atmospheric pressure and the reaction time is ranging from 1 hour to 5 hours.

The present invention also discloses a novel salt of 2-(amino) ethyl methyl sulfone and the preparation method thereof.

The said novel salt of 2-(amino) ethyl methyl sulfone has the following chemical structure: 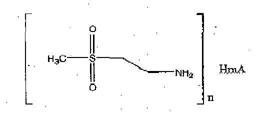 0=1∼3
m=1∼3
HmA= methanoic acid or substituted carboxylic acids, phosphoric acid, sulfonic acid or substituted sulfonic acids such as para-toluenesulfonic acid, sulfuric acid, carbonic acid, boric acid or substituted boric acid, other halogen acids except hydrogen chloride, etc. The said novel salt of 2-(amino) ethyl methyl sulfone could be carboxylate, phosphate, dihydric phosphate, monohydric phosphate, sulphonate, sulphate, bisulfate, carbonate, bicarbonate, borate, other halogen acid salts except hydrochloride, etc. The novel salt of 2-(amino) ethyl methyl sulfone such as 2-(amino)- ethyl methyl sulfone methylbenzenesulfonate has the advantage of being easily purified compared to 2-(amino) ethyl methyl sulfone hydrochloride.

The preparation method of novel salts of 2-(amino) ethyl methyl sulfone is selected from any of the following methods:
A. preparing for 2-(amino) ethyl methyl sulfone using 2-(N,N- dibenzylamino) ethyl methyl sulfone via catalytic hydrogenation and then performing the direct reaction between 2-(amino) ethyl methyl sulfone and acids in organic solvent, or
B. transforming 2-(amino) ethyl methyl sulfone hydrochloride into free alkali using organic or inorganic alkali in organic solvent, and then performing the reaction between free alkali and acids to form corresponding salts.

The said organic or inorganic alkali could be selected from the group consisting of triethylamine, ammonia and hydroxide, etc.

The said organic solvent is selected from the group consisting of alcohols, ethers, substituted amides, etc.

Salts with different acid radical will be obtained by transforming 2-(amino) ethyl methyl sulfone hydrochloride into free alkali in alkali solution of organic solvent and then adding different kinds of acid.

The process of the preparation method is as following: 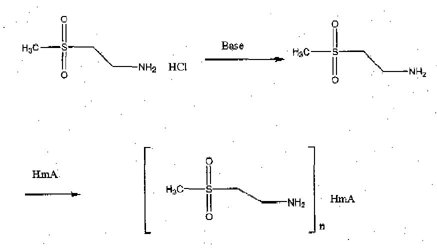 n=1∼3
m=1∼3
HmA= methanoic acid or substituted carboxylic acids, phosphoric acid, sulfonic acid or substituted sulfonic acids, sulfuric acid, carbonic acid, boric acid or substituted boric acid, other halogen acids except hydrogen chloride, etc.

Different salts of 2-(amino) ethyl methyl sulfone will be obtained respectively from such reactions above.

The above substituting reaction could be accomplished at the room temperature and atmospheric pressure.

Furthermore, the said 2-(amino) ethyl methyl sulfone hydrochloride is prepared using 2-(N,N- dibenzylamino) ethyl methyl sulfone as starting material.

The present invention is not only advantageous for reducing environmental pollution, but also could be used for mass fabrication of 2-(amino) ethyl methyl sulfone and the salts thereof, and in addition, the present invention is advantageous for the preparation of the highly purified 2-(amino) ethyl methyl sulfone hydrochloride.

### Detailed Description of the Preferred Embodiment

### Example 1: preparation of 2-methylsulfonyl ethanamine

200ml of diethyl ether and 50g of 2-(methylsulfonyl)ethyl 4-methylbenzenesulfonate were added into a 1000ml stainless steel vessel under stirring, then ammonia was added by an inlet into the vessel until the inner pressure of the vessel reached about 10 kilograms. The reaction was proceeded at 50°C for 1 hour under stirring. Then the temperature was reduced and the reaction was ended. Excessive ammonia was released. The 2'-(methyl sulfone) ethanamine product would be obtained after discharging, staying overnight, filtering and concentration. The oil component could be used for the salt-forming reaction in the next step.

### Example 2: preparation of methylsulfonyl ethanamine hydrochloride

2'-(methyl sulfonyl) ethanamine prepared in example 1 was dissolved in 100ml of alcohol, then hydrogen chloride solution of ethanol was added under stirring until white solid precipitated. 13g of 2'-(methyl sulfonyl) ethanamine hydrochloride with the purity of higher than 98.5% and the melting point ranging from 165.1°C to 167°C would be obtained after filtering and drying. NMR(nuclear magnetic resonance) analysis result was consistent with the expected structure. The ¹HNMR analysis result was as following: 1HNMR(DMSO-d6):8.44(bs,3H), 3.50(m,2H), 3.17(m, 2H), 3.09(s,3H).

### Example 3: preparation of 2-(N,N-dibenzylamino) ethyl methyl sulfone

139g of 2-(methylsulfonyl)ethyl 4-methylbenzenesulfonate, 380ml of chloroform and 5 IF of triethylamine were added in turn into a 1000ml four necked flask. Then after being cooled by cold water bath, 98g of dibenzylamine was added drop by drop using a balancing funnel and the inner temperature was below 35°C and the whole reaction process was traced by thin layer chromatography (TLC) until the starting material 2'-(methyl sulfone) ethyl methylbenzenesulfonate disappeared. The reaction solution was transferred into a 1000nit separatory funnel and washed with 200ml water twice. The organic layer was dried by anhydrous sodium sulfate overnight. After filtration, the filter cake was washed by a few amount of chloroform and 107g of oil component was obtained after concentration of the filtrate. After recrystallization using ethanol, 92g of white crystal 2-(N,N-dibenaylamino) ethyl methyl sulfone was obtained with the melting point ranging from 59°C to 60°C. NMR (nuclear magnetic resonance) analysis result was consistent with the expected structure. The NMR analysis result is as following: ¹HNMR(CDCl3):7.33(bs, 10H), 3.64(s, 3H), 3.03(bs, 4H), 2.75(s, 3H).

### Example 4: prepatation of 2-(amino) ethyl methyl sulfone

400ml of ethanol was added into a 1L stainless steel vessel and then 60g of Z-(N,N-dibenzylarnino) ethyl methyl sulfone obtained in example 3 and 15g of Raney Ni was added, then the vessel was filled with hydrogen until the inner pressure of the vessel was up to about 20 kilograms and the reaction was proceeded at SO°C under stirring, and the whole reaction process was traced by thin layer chromatography (TLC) until the starting material disappeared. The 2-(amino) ethyl methyl sulfone oil component would be obtained after filtering and concentration. The oil component could be used for the salt-forming reaction in the next step.

### Example 5: preparation of 2-(amino) ethyl methyl sulfone hydrochloride

2-(ammo) ethyl methyl sulfone prepared in example 4 was dissolved in 100ml of absolute ethanol, then the hydrogen chloride solution of ethanolwas was added until the pH value was 2 under stirring for 1 hour. After filtration, the filter cake was washed with ethanol and then 11 g of 2-(amino) ethyl methyl sulfone hydrochloride with the purity of higher than 98.5% and the melting point ranging from 165.3°C to 167°C was obtained after drying. NMR (nuclear magnetic resonance) analysis result of the product obtained was consistent with the NMR analysis result of 2- amino ethyl methyl sulfone hydrochloride obtained in the example 2.

### Example 6: preparation of 2-(amino) ethyl methyl sulfone bisulfate

30L of ethanol and 0.58kg of sodium hydroxide were added into a 50L vessel and the mixture was allowed to stir until sodium hydroxide dissolved. Then the reaction mixture was cooled to 20°C. Then 2.3 kg of 2-(amino) ethyl methyl sulfone hydrochloride was added in batches and the mixture was allowed to stir for 0.5 hours. After filtration, the filter cake was washed with ethanol and then the washing liquor and the filtrate were combined After the solution was cooled to about 10°C, 1.4kg of concentrated sulphuric acid was added in batches and then white solid precipitated. Stirring for 2 hours, 2.6 kg of 2-(amino) ethyl methyl sulfone bisulfate was obtained after filtration and being washed with ethanol. NMR analysis result of the product obtained was consistent with the expected structure.

### Example 7: preparation of 2-(amino) ethyl methyl sulfone sulfate

30L of ethanol and 0.58kg of sodium hydroxide were added into a 50L vessel stirring and the mixture was allowed to stir until sodium hydroxide dissolved. Then the mixture was cooled to 20°C and 2.3 3 kg of 2-(amino) ethyl methyl sulfone hydrochloride was added in batches under stirring for 0.5 hours. After filtration, the filter cake was washed with ethanol and then the washing liquor and the filtrate were combined. After the solution was cooled to about 10°C, 0.7kg of concentrated sulphuric acid was added in batches and then white solid precipitated. Stirring for 2 hours, 2.0 kg of 2-(amino) ethyl methyl sulfone sulfate was obtained after filtration and being washed using ethanol. NMR analysis result of the product obtained was consistent with the expected structure.

### Example 8: preparation of 2-(amino) ethyl methyl sulfone methylbenzenesulfonate

The oil component 2-(amino) ethyl methyl sulfone obtained in example 4 was dissolved in 200ml of absolute ethanol and then 35g of p-toluenesulfonic acid monohydrate was added. The mixture was heated to reflux and the solution became clear. Then the mixture was cooled to ambient temperature and placed in the refrigerator overnight afterwards. After filtration and being washed using ethanol, 51 grams of acicular crystal with purity of higher than 98,5% and the melting point ranging from 135 °C to 137°C was obtained, The NMR analysis result was as following: ¹HNMR(DMSO-d6):7.86(bs,3H), 7.44 (d,2H) ,7.07(d, 2H),3.38(m,2H), 3.24(m, 2H), 3.10(s,3H),2.27(s,3H).

## Claims

1. A novel preparation method of sulfonyl alkylamine and the salts thereof including the steps as following:
A. adopting sulfonates containing a sulfone moiety having the following general formula as starting material: 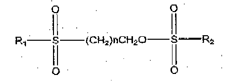 R₁=alkyl
R₂=Alkyl, Aryl
n=1∼18
B: adopting ammonia or amine having the following general formula as substituting agent to react with the starting material: 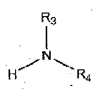 R₃=H, Methyl, Ethyl, n-Propyl
R₄=H,(CH₂)nR₅ R₅=Alkyl, Aryl, etc n=0 or 1.

2. The novel preparation method of sulfonyl alkylamine and the salts thereof, as recited in claim 1, wherein in step B, ammonia or amine is used as the substituting agent to react with the starting material to prepare for the sulfonyl alkylamine and the process is as following:

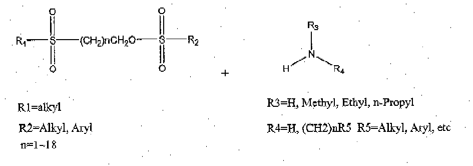

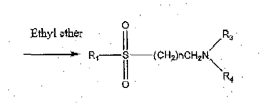

3. The novel preparation method of sulfonyl alkylamine and the salts thereof, as recited in claim 2, wherein the sulfonyl alkylamine obtained in step B could be further adopted to react with acids to form salts via the salt-forming reaction.

4. The novel preparation method of sulfonyl alkylamine and the salts thereof, as recited in claim 3, wherein the solvent adopted in the salt-forming reaction is selected from a group consisting of linear or cyclic ethers, alcohols, etc.

5. The novel preparation method of sulfonyl alkylamine and the salts thereof, as recited in claim 1, wherein the solvent in step B is selected from the group consisting of ammonia, amine, the organic solution of ammonia and the organic solution of amine, the ratio of sulfonates containing a sulfone moiety and the substituting agent is like that: the amount of the ammonia or amine is more than twice of the theory amount.

6. The novel preparation method af sulfonyl alkylamine and the salts thereof, as recited in claim 1, wherein 2-(methylsulfonyl)ethyl 4-methylbenzenesulfonate having the following structure is adopted as the starting material in step A: 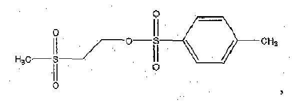 and N,N-dibmzylamine having the following structure is adopted as the substituting agent in step B: 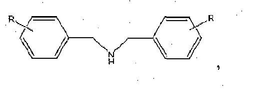 and the process of the reaction used for the preparation of 2-(N,N- dibenzylamino) ethyl methyl sulfone and salts thereof is as following: 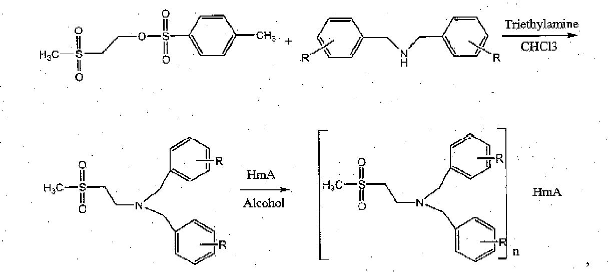 the product obtained is 2-(N,N- dibenzylamino) ethyl methyl sulfone and salts thereof having the following structure: 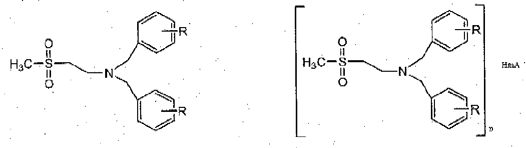 R=H, halogen, alkoxy, etc.
m=1-3, n=3-1
HmA= hydrochloric acid, sulfuric acid, phosphoric acid, etc.

7. A novel 2-(N,N- dibenzylamino) ethyl methyl sulfone and the salts thereof having the following st1Ucture: 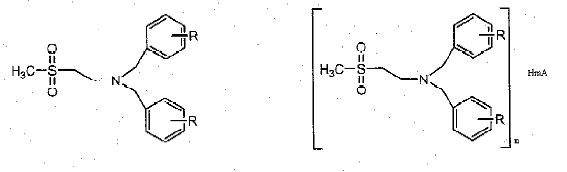 R=H, halogen, alkoxy, etc.
m=1-3,
n=3-1
HmA= hydrochloric acid, sulfuric acid, phosphoric acid, etc.

8. The use of 2-(N,N- dibenzylamino) ethyl methyl sulfone and the salts thereof, as recited in claim 7, for the preparation of 2-(amino) ethyl methyl sulfone and the salts thereof.

9. A preparation method of 2-(amino) ethyl methyl sulfone and the salts thereof, wherein the said 2-(amino) ethyl methyl sulfone and the salts thereof are prepared via catalytic hydrogenation of 2-(N,N-dibenzylamino) ethyl methyl sulfone and the salts thereof as recited in claim 7 and the catalytic agent is selected from a group consisting of Raney Ni, Pd/C and platinum dioxide.

10. The preparation method of 2-(amino) ethyl methyl sulfone and the salts thereof as recited in claim 9, wherein the process of the preparation method is as following: 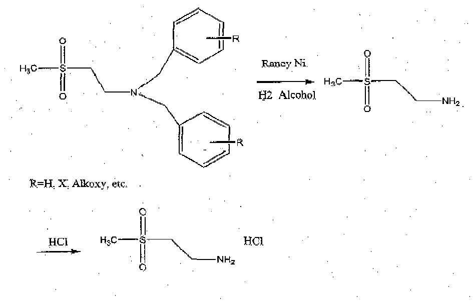 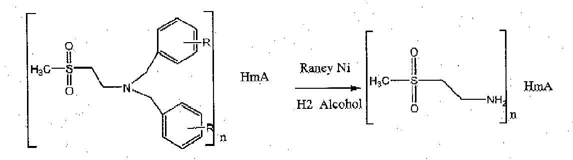 R=H, X, Alkoxy, etc.
m=1-3, n=3-1.

11. A novel salt of 2-(amino) ethyl methyl sulfone having the following structure: 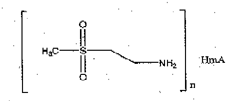 n=1∼3
m=1∼3
HmA= methanoic acid or substituted carboxylic acids, phosphoric acid, sulfonic acid or substituted sulfonic acids, sulfuric acid, carbonic acid, boric acid or substituted boric acid, other halogen acids except hydrogen chloride, etc.

12. The novel salt of 2-(amino) ethyl methyl sulfone, as recited in claim 11, wherein the said novel salt of 2-(amino) ethyl methyl sulfone is prepared via the process consisting the steps of preparing for free 2-(amino) ethyl methyl sulfone firstly and then performing the direct reaction between 2-(amino) ethyl methyl sulfone and acids to form the novel salt of 2-(amino) ethyl methyl sulfone.

13. A preparation method of the novel salt of 2-(amino) ethyl methyl sulfone as recited in claim 11 or 12, wherein the preparation method is selected from any of the following methods:
A, preparing for 2-(amino) ethyl methyl sulfone using 2-(N,N- dibenzylamino) ethyl methyl sulfone as recited in claim 7 via catalytic hydrogenation and then performing the direct reaction between 2-(amino) ethyl methyl sulfone and acids in organic solvent, or
B. transforming 2-(amino) ethyl methyl sulfone hydrochloride into free alkali using organic or inorganic alkali in organic solvent, and then performing the reaction between free alkali and acids to form corresponding salts.

14. The preparation method of the novel salt of 2-(amino) ethyl methyl sulfone, as recited in claim 13, wherein the said organic solvent adopted is selected from the group consisting of alcohols, ethers, substituted amides, etc.

15. The preparation method of the novel salt of 2-(amino) ethyl methyl sulfone, as recited in claim 14, wherein the process of the preparation method B is as following: 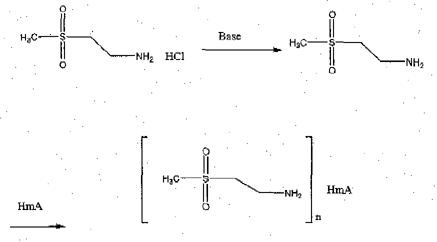 n=1∼3
m=1∼3
HmA= methanoic acid or substituted carboxylic acids, phosphoric acid, sulfonic acid or substituted sulfonic acids such as para-toluenesulfonic acid, sulfuric acid, carbolic acid, boric acid or substituted boric acid, other halogen acids except hydrogen chloride, etc.
